# EUROPEAN PATENT APPLICATION

(11) **EP 4 119 145 A1**
(43) Date of publication of application: **18.01.2023**
(21) Application number: 21185898.0
(22) Date of filing: 15.07.2021
(51) Int. Cl.: A61K 31/529, A61K 31/04, A61K 31/136, A61K 31/155, A61K 31/164, A61K 31/343, A61K 31/352, A61K 31/404, A61K 31/428, A61K 31/47, A61K 31/473, A61K 31/55, A61K 31/7056, A61P 31/14

(54) **COMPOUNDS FOR THE TREATMENT OF COVID-19**

(71) Applicant: Dompe' Farmaceutici S.P.A., 20122 Milan (IT); Fraunhofer-Gesellschaft zur Förderung der angewandten Forschung e.V., 80686 München (DE)
(72) Inventor: BECCARI, Andrea Rosario, 80131 Napoli (IT); IACONIS, Daniela, 80131 Napoli (IT); MANELFI, Candida, 80131 Napoli (IT); TALARICO, Carmine, 80131 Napoli (IT); ZALIANI, Andrea, 22359 Hamburg (DE); ZUZIKOV, Maria, 80686 München (DE); GRIBBON, Philip, 22587 Hamburg (DE); GEISSLINGER, Gerd, 60596 Frankfurt am Main (DE)
(74) Representative: Mauri, Elisabetta Maria Ester

(57) **Abstract**

The present invention relates to compounds for the treatment of a SARS-Cov-2 infection in a subject.

## Description

### FIELD OF THE INVENTION

The present invention relates to compounds that are able to inhibit replication of SARS-CoV-2 virus and thus are useful in the treatment of SARS-CoV-2 virus infections.

### STATE OF THE ART

Coronaviruses (Covs) are a large family of single-stranded, enveloped RNA viruses that belong to the Coronaviridae family. The limited number of coronaviruses known to be able to infect humans were considered in the past as relatively harmless respiratory human pathogens, causing mild infections. However, two coronavirus subtypes have emerged, Severe Acute Respiratory Syndrome Coronavirus (SARS-CoV) and the Middle East Respiratory Syndrome Coronavirus (MERS-CoV), which cause severe and sometimes fatal respiratory tract infections in humans (Pereira, H et al, Andrewes' Viruses of Vertebrates 1989, 5th ed., p. 42-57; Holmes K.V., Lai M.M.C. Coronaviridae and their replication. In: Fields B.N., Knipe D.M., Howley P.M., editors. Fields Virology. 3rd edn. Lippincott-Raven; Philadelphia: 1996. p. 1075). In December 2019, atypical pneumonia cases occurred in China and the cause was later identified as being a novel coronavirus. The World Health Organization (WHO) named the virus as SARS-CoV-2 and the related disease as COVID-19.

The virus spread rapidly worldwide, and on 11 March 2020 the WHO declared SARS-CoV-2 infection as a pandemic. Most people infected with COVID-19 experience mild to moderate respiratory illness (fever, fatigue, dry cough and dyspnoea) and recover without requiring special treatments. Older people, and those with underlying medical problems like cardiovascular disease, diabetes, chronic respiratory disease, and cancer are more likely to develop serious illness. Furthermore, analysis of epidemiological and clinical characteristics and outcomes of patients infected by SARS-CoV-2 have shown that 15% of people with symptomatic COVID-19 have significant disease including severe pneumonia, and 5% experience critical disease with life-threatening complications. Critical disease includes acute respiratory distress syndrome (ARDS), sepsis, septic shock, cardiac disease, thromboembolic events, such as pulmonary embolism and multi-organ failure.

There is also growing evidence that, in those who develop critical COVID-19 disease, long-term consequences such as rare neurological and psychiatric complications are reasonably expected. These may include stroke, delirium, anxiety, depression, damage or inflammation of the brain and sleep disturbances.

Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Dong, N. et al., F1000Research 2020, 9: 121). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and also uses the same cell entry receptor, Angiotensin-converting enzyme II (ACE2).

In particular, the spike protein at the surface of the virus binds to ACE2 through its receptor-binding domain (RBD), thereby enabling the entry of the virus into cells. After entry into the host cells, the viral RNA is released into the cytoplasm and is then translated in two polyproteins, pp1a and pp1b, that are cleaved into nonstructural proteins NSP1-16 by the viral proteases papain-like protease (PLpro) and 3C-like protease (3CL-Pro) (Moustaqil et al, Emerging Microbes & Infections 2021, 10(1), 178-195). NSPs have important roles in replication and transcription (Rajarshi et al, Gene 2021, 768: 145313). PLpro and 3CL-Pro are also responsible for cleaving mediators of host antiviral immune response, thus providing a mechanism by which the virus evades the immune system (Choudhury et al, In Silico Pharmacol 2021, 9: 26; Liu et al, Viruses 2020, 12: 1039).

Therefore, the above SARS-CoV-2 proteins represent effective therapeutic targets in SARS-CoV-2 for preventing replication and proliferation of the virus.

The identification and development of new drugs is a lengthy process that is not thus adequate to face the emergency of the immediate global challenge of COVID-19 outbreak. Repurposing of drugs already tested as safe in man or approved for different therapeutic is a rapid response solution, since the pharmacokinetic, toxicological, and manufacturing data for the drigs are already available, thus allowing immediate application in clinical setting (Anand et al., Science 2003;300(5626):1763-7).

### SUMMARY OF THE INVENTION

The applicants have carried out the analysis of a library containing more than 10,000 commercialized drugs and clinical candidates "safe in man" or characterized up to late clinical stage and have selected by Computer-Aided Drug Design a number of molecules able to bind to SARS-Cov-2 functional proteins essential for the pathogenetic mechanisms. Furthermore, the applicants have confirmed by in vitro screening the ability of the selected molecules to inhibit virus survival and replication in infected host cells.

In details, the compounds selected are better described below, wherein also the chemical name and formula for each compound are provided.

### Compounds of formula (I)

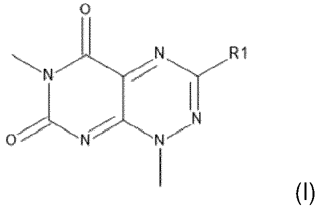

wherein
R1 is selected from p-trifluoromethylphenyl and CH₃;

### compounds of formula (II)

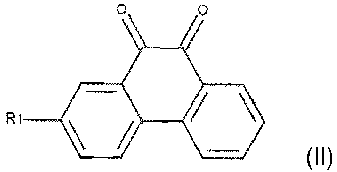

wherein
R1 is selected from the group consisting of H, NO₂, NH₂, and NHCOC(CH₃)₃; the compounds listed in the table below:

| **N.** | **Chemical structure** | **Current Name** | **Chemical Name** |
|---|---|---|---|
| 3 | 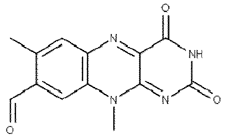 | Ro-08-2750 | 7,10-Dimethyl-2,4-dioxo-2,3,4,10-tetrahydrobenzo[g]pteridine-8-carbaldehyde |
| 8 | 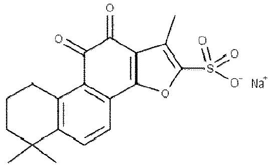 | Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| 9 | 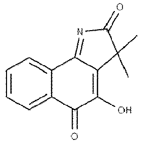 | BVT-948 | 4-Hydroxy-3,3-dimethyl-3,5-dihydro-2H-benzobenzo[g]indole-2,5-dione |
| 10 | 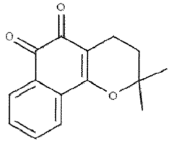 | beta-Lapachone | 2,2-Dimethyl-3,4-dihydro-2H-benzo[h]1-benzopyrane-5,6-dione |
| 11 | 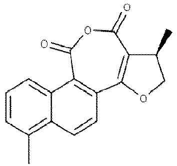 | Dihydrotanshinone | (4R)-4,9-Dimethyl-4,5-dihydrofuro[3,2-c]naphtho[2,1-e]oxepine-1,3-dione |
| 12 | 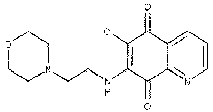 | NSC-663284 | 6-Chloro-7-[2-(4-morpholinyl)ethylamino]-5,8-dihydroquinoline-5,8-dione |
| 13 | 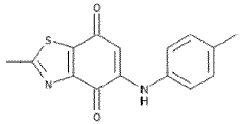 | Ryuvidine | 2-methyl-5-(4-methylanilino)-1,3-benzothiazole-4,7-dione |
| 14 | 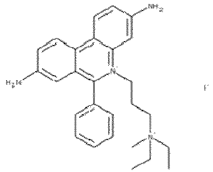 | Propidium Iodide | 3,8-Diamino-5-(3-(diethyl(methyl)ammonio)propyl) -6-phenylphenanthridin-5-ium iodide |
| 15 | 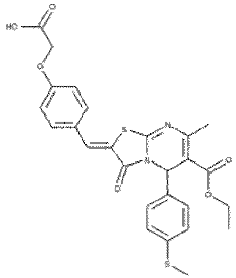 | ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| 16 | 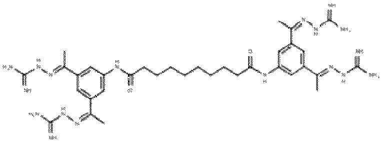 | SEMAPIMOD | N,N'-Bis[3,5-bis[1-(2-amidinohydrazono)ethyl]phenyl] decanediamide |
| 17 | 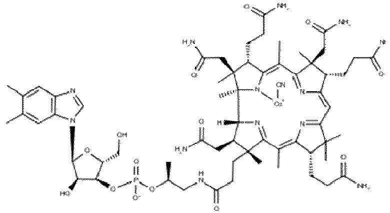 | Cyanocobalamin | cobalt(3+);[(2R,3S,4R,5S)-5-(5,6-dimethylbenzimidazol-1-yl)-4-hydroxy-2-(hydroxymethyl)oxolan-3-yl] [(2R)-1-[3-[(1R,2R,3R,4Z,7S,9Z,12S,13S,14Z, 17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl]propanoylamino]propan-2-yl] phosphate;cyanide |

All these compounds are well known approved drugs or clinical candidates for different therapeutic indications with "safe in man" trials completed.

The inventors have also tested pharmaceutically acceptable salts and hydrate forms of the above compounds and have confirmed that these also maintain the activity against the virus.

The experimental results obtained by the inventors and described in the Experimental Section below demonstrate that all these compounds are useful for the treatment of a SARS-Cov-2 infection in a subject.

Accordingly, a first object of the invention is a compound selected from the compounds above and pharmaceutically acceptable salt thereof, for use in the treatment of a SARS-Cov-2 infection in a subject.

A second object of the invention is a pharmaceutical composition comprising i) at least one compound according to the first object of the invention or pharmaceutically acceptable salts thereof, and ii) at least one inert pharmaceutically acceptable excipient, for use in the treatment of a SARS-Cov-2 infection in a subject.

A third object of the invention is a method of treating a SARS-Cov-2 infection in a subject, comprising administering to the subject at least one compound according to the first object of the invention or pharmaceutically acceptable salts therof.

### DETAILED DESCRIPTION OF THE INVENTION

A first object of the present invention is a compound for use in the treatment of a SARS-Cov-2 infection in a subject, wherein said compound is selected from:
a compound of formula (I) 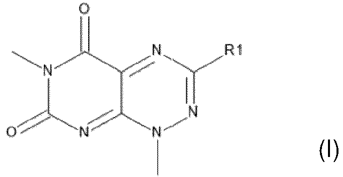 wherein
R1 is selected from p-trifluoromethylphenyl and CH₃;
a compound of formula (II) 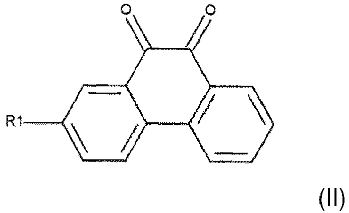 wherein
R1 is selected from the group consisting of H, NO₂, NH₂, and NHCOC(CH₃)₃; and the compounds listed in the table below:

| **N.** | **Chemical structure** | **Current Name** | **Chemical Name** |
|---|---|---|---|
| 3 | 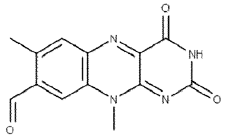 | Ro-08-2750 | 7,10-Dimethyl-2,4-dioxo-2,3,4,10-tetrahydrobenzo[g]pteridine-8-carbaldehyde |
| 8 | 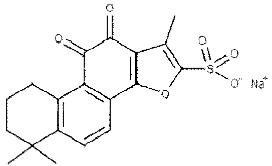 | Sodium tanshinone II-A sulfonate | 1,6,6-Tri methyl-10,11-dioxo-6,7,8,9,10,11-hexahydrophenanthro[1,2-b]furan-2-sulfonic acid sodium salt |
| 9 | 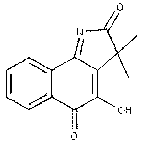 | BVT-948 | 4-Hydroxy-3,3-dimethyl-3,5-dihydro-2H-benzobenzo[g]indole-2,5-dione |
| 10 | 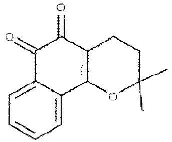 | beta-Lapachone | 2,2-Dimethyl-3,4-dihydro-2H-benzo[h]1-benzopyrane-5,6-dione |
| 11 | 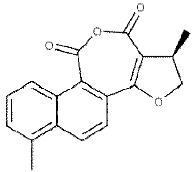 | Dihydrotanshinone | (4R)-4,9-Dimethyl-4,5-dihydrofuro[3,2-c]naphtho[2,1-e]oxepine-1,3-dione |
| 12 | 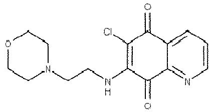 | NSC-663284 | 6-Chloro-7-[2-(4-morpholinyl)ethylamino]-5,8-dihydroquinoline-5,8-dione |
| 13 | 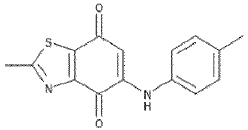 | Ryuvidine | 2-methyl-5-(4-methylanilino)-1,3-benzothiazole-4,7-dione |
| 14 | 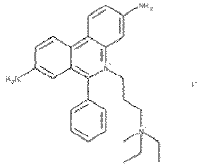 | Propidium Iodide | 3,8-Diamino-5-(3-(diethyl(methyl)ammonio)propyl)-6-phenylphenanthridin-5-ium iodide |
| 15 | 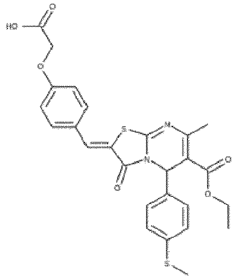 | ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| 16 | 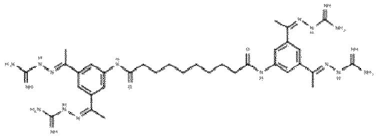 | SEMAPIMOD | N,N'-Bis[3,5-bis[1-(2-amidinohydrazono)ethyl]phenyl] decanediamide |
| 17 | 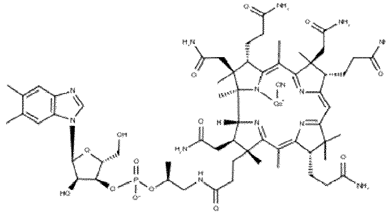 | Cyanocobalamin | cobalt(3+);[(2R,3S,4R,5S)-5-(5,6-dimethylbenzimidazol-1-yl)-4-hydroxy-2-(hydroxymethyl)oxolan-3-yl][(2R)-1-[3-[(1R,2R,3R,4Z,7S,9Z,12S,13S,14Z,17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl]propanoylamino]propan-2-yl] phosphate;cyanide |

and pharmaceutically acceptable salts thereof.

In a preferred embodiment, said compound of formula (I) is selected from 1,6-Dimethyl-3-[4-(trifluoromethyl)phenyl]-1,5,6,7-tetrahydropyrimido[5,4-e][1,2,4]triazine-5,7-dione, 1,3,6-Trimethylpyrimido[5,4-e][1,2,4]triazine-5,7(1H,6H)-dione and pharmaceutically acceptable salts thereof.

In a preferred embodiment, said compound of formula (II) is selected from the group consisting of 2-Nitrophenanthrene-9,10-dione, N-(9,10-Dioxo-9,10-dihydrophenanthren-2-yl)-2,2-dimethylpropionamide, phenanthrene-9,10-dione, 2-aminophenanthrene-9,10-dione and pharmaceutically acceptable salts thereof.

In a preferred embodiment, the compound for use according to the present invention is selected from the compounds listed in the table below:

| **N.** | **Chemical structure** | **Current Name** | **Chemical Name** |
|---|---|---|---|
| 1 | 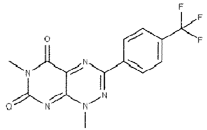 | Walrycin B | 1,6-Dimethyl-3-[4-(trifluoromethyl)phenyl]-1,5,6,7-tetrahydropyrimido[5,4-e][1,2,4]triazine-5,7-dione |
| 2 | 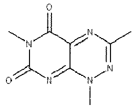 | 3-Methyltoxoflavin | 1,3,6-Trimethy!pyrimido[5,4-e][1,2,4]triazine-5,7(1H,6H)-dione |
| 3 | 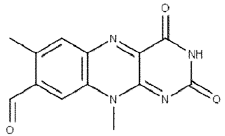 | Ro-08-2750 | 7,10-Dimethyl-2,4-dioxo-2,3,4,10-tetrahydrobenzo[g]pteridine-8-carbaldehyde |
| 4 | 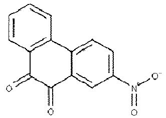 | NSC-23180 | 2-Nitrophenanthrene-9,10-dione |
| 5 | 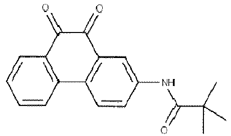 | SF1670 | N-(9,10-Dioxo-9,10-dihydrophenanthren-2-yl)-2,2-dimethylpropionamide |
| 6 | 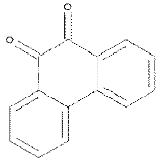 | 9,10-Phenanthrenequin one | phenanthrene-9,10-dione |
| 7 | 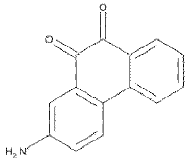 | PD086278 | 2-aminophenanthrene-9,10-dione |
| 8 | 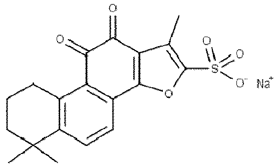 | Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| 9 | 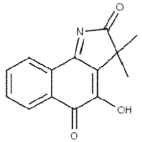 | BVT-948 | 4-Hydroxy-3,3-dimethyl-3,5-dihydro-2H-benzobenzo[g]indole-2,5-dione |
| 10 | 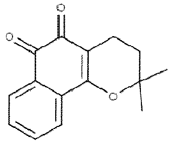 | beta-Lapachone | 2,2-Dimethyl-3,4-dihydro-2H-benzo[h]1-benzopyrane-5,6-dione |
| 11 | 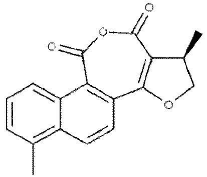 | Dihydrotanshinone | (4R)-4,9-Dimethyl-4,5-dihydrofuro[3,2-c]naphtho[2,1-e]oxepine-1,3-dione |
| 12 | 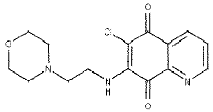 | NSC-663284 | 6-Chloro-7-[2-(4-morpholinyl)ethylamino]-5,8-dihydroquinoline-5,8-dione |
| 13 | 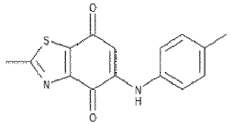 | Ryuvidine | 2-methyl-5-(4-methylanilino)-1,3-benzothiazole-4,7-dione |
| 14 | 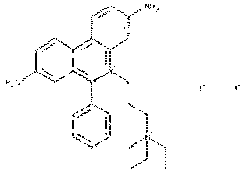 | Propidium Iodide | 3,8-Diamino-5-(3-(diethyl(methyl)ammonio)propyl)-6-phenylphenanthridin-5-ium iodide |
| 15 | 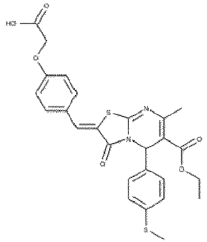 | ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| 16 | 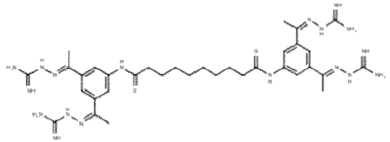 | SEMAPIMOD | N,N'-Bis[3,5-bis[1-(2-amidinohydrazono)ethyl]phenyl]decanediamide |
| 17 | 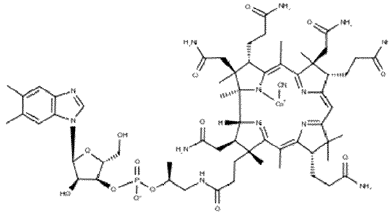 | Cyanocobalamin | cobalt(3+);[(2R,3S,4R,5S)-5-(5,6-dimethylbenzimidazol-1-yl)-4-hydroxy-2-(hydroxymethyl)oxolan-3-yl] [(2R)-1-[3-[(1R,2R,3R,4Z,7S,9Z,12S,13S,14Z, 17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl]propanoylamino]propan-2-yl] phosphate;cyanide |

and pharmaceutically acceptable salts thereof.

The compound according to the first object of the invention may be administered alone or as a combination of compounds according to the first object of the invention. According to the latter, preferably a combination of two compounds selected from compounds n. 1 to 17 in the table above can be used in combined therapy.

The compounds of the combination can be administered simultaneously or sequentially. Preferably, when the compounds are administered simultaneously, they are both present in the same pharmaceutical formulation.

The identification of a SARS-Cov-2 infection in a subject is usually based on the analysis of the presence of viral RNA in biological material from the subject, preferably specimens from upper and lower respiratory tract, such as nasopharyngeal/oropharyngeal swabs, nasal aspirate, nasal wash or saliva, sputum, tracheal aspirate or bronchoalveolar lavage. The subject to be treated may have one or more symptoms of COVID-19 or be asymptomatic.

As used herein the term "pharmaceutically acceptable salts" of the compounds according to the first object of the invention refers to addition salts of these compounds with acids or bases that form non-toxic acid anions or cations.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

The salts may be formed by conventional means, such as by reacting the free form of the compound with one or more equivalents of the appropriate acid or base in a solvent or medium in which the salt is insoluble, such as for example water or ethanol, which is removed under vacuum or by freeze drying.

The compounds according to the first object of the invention may be in an anhydrous or a hydrate form.

The present invention also includes prodrugs of the compounds according to the first object of the invention.

As used herein, the term "prodrug" refers to a biologically inactive compound which can be metabolized in the body to produce a compound according to the first object of the invention. Examples, without limitation, of prodrugs are ester of the compounds according to the first object of the invention, that facilitate transmittal across a cell membrane where water solubility is not beneficial, but then are metabolically hydrolysed once inside the cell where water solubility is beneficial.

The compound according to the first object of the invention is administered in form of a pharmaceutical composition formed by admixture of the compound with one or more pharmaceutically acceptable excipients.

Thus, a second object of the invention is a pharmaceutical composition comprising at least one compound according to the first object of the invention or pharmaceutically acceptable salts thereof, and at least one inert pharmaceutically acceptable excipient, for use in the treatment of a SARS-Cov-2 infection in a subject.

According to a preferred emobodiment, the pharmaceutical composition comprises one compound according to the first object of the invention.

According to an alternative embodiment, the pharmaceutical composition comprises a combination of compounds according to the first object of the invention selected from compounds n. 1 to 17 in the table above and at least one inert pharmaceutically acceptable excipient, for use in the treatment of a SARS-CoV-2 infection in a subject. The route of administration of the compound or pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, and intracutaneous or intralesional injection or infusion techniques.

In one preferred embodiment, the invention provides for administration of the compound or the pharmaceutical composition of the invention such that a sufficient concentration of the compound is reached in the respiratory tract. The skilled person is able to determine such sufficient concentration in easily and routinely manner based on the compound or pharmaceutical composition that is administered.

Preferably, this is obtained by direct administration of the compound or pharmaceutical composition of the present invention to the respiratory tract. The direct administration to the respiratory tract may be the sole route of administration of the compound or composition or may be combined with administration of the compound or composition via other systemic routes.

For direct administration of the compound or composition of the invention, a wide variety of devices that are designed for the direct delivery of pharmaceutical compositions and therapeutic formulations to the respiratory tract may be used. In one aspect of the present invention, the compound or pharmaceutical composition of the present invention is administered in aerosolized or inhaled form.

The pharmaceutical composition of the present invention for direct administration to the respiratory tract as described above can be in form of an aerosol formulation, as a dry powder or a solution or suspension with a diluent.

Preferably, the pharmaceutically acceptable excipient includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, and salts for regulating osmotic pressure, emulsifiers, sweeteners, colorants, flavourings and the like.

The amount of said compound in the pharmaceutical composition of the present invention is the amount usually employed for other indications of the specific compound.

The amount can vary over a wide range depending on known factors, for example the molecule used, the severity of the disease, the patient's body weight, the dosage form, the chosen route of administration, and the number of administrations per day. However, a person skilled in the art can determine the optimum amount in easily and routinely manner.

Also specific dosage and treatment regimens for any particular patient will depend upon a variety of factors, including for example the activity and half life of the specific compound employed, the age, body weight, general health status, sex, diet, severity and course of the disease.

The dosage forms of the pharmaceutical composition of the present invention can be prepared by techniques that are familiar to a pharmaceutical chemist, and comprise mixing, granulation, compression, dissolution, sterilization and the like.

A third object of the invention is a method of treating a SARS-Cov-2 infection in a subject, comprising administering to said subject at least one compound according to the first object of the invention or pharmaceutically acceptable salts thereof or a pharmaceutical composition according to the second object of the invention.

According to a preferred emobodiment, said method involves administering one compound according to the first object of the invention.

According to an alternative embodiment, said method involves administering a combination of compounds according to the first object of the invention. According to the latter, preferably the method involves administering a combination of two compounds selected from compounds n. 1 to 17 in the table above or pharmaceutically acceptable salts thereof. The compounds of the combination can be administered simultaneously or sequentially.

The invention will be further described in the following examples, which do not limit the scope of the invention described in the claims.

### EXPERIMENTAL PART

Compound repurposing is an important strategy for the identification of effective treatment options against SARS-CoV-2 infection and COVID-19 disease. As a single-stranded positive-sense RNA virus, SARS-CoV-2 uses cellular translation machinery directly after infection of the cell to produce viral polyproteins pp1a and pp1ab. The generation of individual viral proteins involves proteolytic cleavage by 3CL-Pro and PLpro (Wu C, et al., Analysis of therapeutic targets for SARS-CoV-2 and discovery of potential drugs by computational methods. Acta Pharm Sin B. 2020 May;10(5):766-788). PLpro protease is also involved in a more important function, the suppression of the host innate immune response through deubiquitination and delSGylation of the proteins involved in the antiviral immune response (Petushkova et al., Papain-Like Proteases as Coronaviral Drug Targets: Current Inhibitors, Opportunities, and Limitations. Pharmaceuticals (Basel). 2020 Sep 28;13(10):277). Because of its role both in the viral replication and in the immune suppression, PLpro represents an attractive target for antiviral drug development (Shen et al., Potent, Novel SARS-CoV-2 PLpro Inhibitors Block Viral Replication in Monkey and Human Cell Cultures. bioRxiv [Preprint]. 2021 Feb 15:2021.02.13.431008). The Applicants therefore undertook studies in order to identify compounds able to target PLpro.

### EXAMPLE 1

### Computer-Aided Drug Design

The sequence and crystal structure of the PL protease of SARS-CoV-2 was obtained from the Protein Data Bank with the code 6W9C (https://www.rcsb.org/structure/6w9c).

The EXSCALATE platform, the most powerful computing resources currently based in Europe to empower smart in-silico drug design (co-funded by the H2020-FET-HPC ANTAREX project), was exploited to perform molecular dynamics (MD) and docking simulations, with the final aim to select and make available molecules active against the PL protease of SARS-CoV-2. Molecular dynamics simulations on the PL protease of SARS-CoV-2 were performed to explore the conformational space of the active site of the protease, and select several protein conformations particularly suitable for docking simulations.

In the first step, MD simulations were carried out on the structure of the PL protease of SARS-CoV-2, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

In a second step, High Performance Computing (HPC) simulation was conducted to virtual screen against the PL protease of SARS-CoV-2 the Safe in Man (SIM) library, containing commercialized and under development drugs, already proved safe in man (> 10,000 drugs), and the Fraunhofer's BROAD Repurposing Library, containing 5400 marketed drugs and clinical stage compounds and molecules with known mode of action. Duplicates between the libraries were removed before ligand preparation, where all compounds were converted to 3D and prepared with Schrödinger's LigPrep tool. This process generated multiple states for stereoisomers, tautomers, ring conformations (1 stable ring conformer by default) and protonation states. In particular, another Schrödinger package, Epik, was used to assign tautomers and protonation states that would be dominant at a selected pH range (pH=7±1). Ambiguous chiral centers were enumerated, allowing a maximum of 32 isomers to be produced from each input structure. Then, an energy minimization was performed with the OPLS3 force.

The simulation was performed using LiGen^{™} (Ligand Generator), the de novo structure based virtual screening software, designed and developed to run on HPC architectures, which represent the most relevant tool of the EXSCALATE platform. LiGen^{™} is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen, the pharmacophoric docking (LiGenPh4) and the geometrical docking (LiGenGeodock), as well as the different scoring functions calculated, were used in this study to explore different protocols of Virtual Screening (VS) and select the best one in terms of performance. The performances of the tested VS strategy were assessed by evaluating the capacity to correctly rank a set of active compounds. In particular, to this purpose, 100 known inhibitors of SARS-CoV-1 3C-like protease were included in the screened database and are considered as actives in the docking studies representing the training set in the optimization/validation analyses.

When tautomers were involved, only the one with the best docking score was chosen. The docking score values that predict the binding affinity of the molecules in the protein binding site are reported (the higher, the better).

Table 1 reports the results obtained for the best scored molecules, selected for further validation of the activity against the PL protease of SARS-CoV-2 in in vitro experiments. In addition to the compounds reported in Table 1, also the compound Cyanocobalamin was tested in in vitro experiments.

**Table 1**

| **N.** | **Current Name** | **Docking Score** |
|---|---|---|
| 1 | Walrycin B | 5.55 |
| 2 | 3-Methyltoxoflavin | 4.79 |
| 3 | Ro-08-2750 | 5.36 |
| 4 | NSC-23180 | 5.38 |
| 5 | SF1670 | 5.78 |
| 6 | 9,10-Phenanthrenequinone | 5.27 |
| 7 | PD086278 | 5.28 |
| 8 | Sodium tanshinone II-A sulfonate | 5.97 |
| 9 | BVT-948 | 5.35 |
| 10 | beta-Lapachone | 5.47 |
| 11 | Dihydrotanshinone | 5.74 |
| 12 | NSC-663284 | 5.06 |
| 13 | Ryuvidine | 5.66 |
| 14 | Propidium Iodide | 6.02 |
| 15 | ML120 | 6.01 |
| 16 | SEMAPIMOD | 6.43 |

### EXAMPLE 2

### In vitro screening of activity against SARS-CoV-2: PLpro biochemical assay

The compounds selected by the CADD analysis and Cyanocobalamin were tested on a FRET based biochemical assay, in accordance with recent publications by Shin et al., 2020 (Shin, D., Mukherjee, R., Grewe, D. et al. Papain-like protease regulates SARS-CoV-2 viral spread and innate immunity. Nature 587, 657-662 (2020)) to validate their ability of inhibiting PLpro protease activity.

We used a commercial source of protein (BPS Bioscience #100735) and a fluorescently labeled ISG15 as a substrate (BostonBiochem ISG15/UCRP AMC, #UL-553). The assay was performed in a buffer containing 50 mM Tris (pH 7.5) and 150 mM NaCl, using 100 nM of SARS-CoV-2 protein and 2.5 µM FRET-substrate.

Firstly, we performed a primary screen in this conditions: 0.15 µM substrate; 1 nM SARS-CoV-2 PL-Pro; 20 µM compound; and 0.2 % DMSO in a total volume of 10 µL/well diluted in the assay buffer (50 mM Tris, 150 mM NaCl, 1 mM DTT, 0.01 % Tween20, pH 7.5). 20 µM PR-619 is used as positive control whereas the same final mix without the compound is used as negative control (Altun M. et al., Activity-based chemical proteomics accelerates inhibitor development for deubiquitylating enzymes. Chem Biol. 2011 Nov 23;18(11):1401-12). The compounds were pre-incubated for 60 min at 25 °C with PLpro to facilitate the identification of slowly binding putative cysteine-reactive inhibitors. The fluorescence signal was then measured at 15 min and inhibition (%) calculated relative to controls (Envision, PerkinElmer). Results were normalized to the 100 % (positive control) and 0 % (negative control) inhibition. To flag possible optical interference effects, primary assay plates were also read 60 min after substrate addition, when the reaction was complete. In this experimental set, some compounds used as positive controls slow down or lose their inhibition activity in the presence of DTT. Therefore, to account for any DTT dependent effects, primary screening on the positive control was performed without DTT in the assay buffer.

Furthermore, the compounds were profiled in dose response achieved with serial dilution at the following concentration points: 0.014 µM, 0.041 µM, 0.122 µM, 0.370 µM, 1.111 µM, 3.333 µM, 6.667 µM, 20 µM.

To characterize the compounds, we evaluated the following parameters:
Inhibition (%): This parameter is strictly linked to fluorescence signal registered during the assay. Given that positive and negative control fluorescence is considered 100% and 0%, respectively; the fluorescence registered in compounds wells is reported to this scale.
IC₅₀ (µM): indicates the half maximal concentration needed to reach the maximal value of enzyme inhibition, determined from dose -response curves.

The parameters obtained for the tested compounds are reported in Table 2 below.

**Table 2**

| **N.** | **Current Name** | **IC₅₀ (µM)** | **PLPro Inhibition [%] Primary Screen (20 µM)** |
|---|---|---|---|
| 1 | Walrycin B | 0.038 | 160.54 |
| 2 | 3-Methyltoxoflavin | 0.074 | 181.38 |
| 3 | Ro-08-2750 | 0.401 | 119.29 |
| 4 | NSC-23180 | 0.129 | 186.10 |
| 5 | SF1670 | 0.246 | 183.62 |
| 6 | 9,10-Phenanthrenequinone | 0.504 | 180.94 |
| 7 | PD086278 | 0.807 | 180.40 |
| 8 | Sodium tanshinone II-A sulfonate | 4.67 | 124.17 |
| 9 | BVT-948 | 0.39 | 154.88 |
| 10 | beta-Lapachone | 4.43 | 146.26 |
| 11 | Dihydrotanshinone | 0.92 | 80.74 |
| 12 | NSC-663284 | 0.22 | 150.90 |
| 13 | Ryuvidine | 0.26 | 159.90 |
| 14 | Propidium Iodide | 0.24 | 135.55 |
| 15 | ML120 | 3.14 | 82.78 |
| 16 | SEMAPIMOD | 1.21 | 140.16 |
| 17 | Cyanocobalamin | 2.9 | 141.27 |

All the selected compounds are able to inhibit the PLpro protease activity, showing a percentage of inhibition higher than 80% at 20 µM and an IC₅₀ value lower than 5 µM.

## Claims

1. A compound for use in the treatment of a SARS-Cov-2 infection in a subject, selected from:
a compound of formula (I) 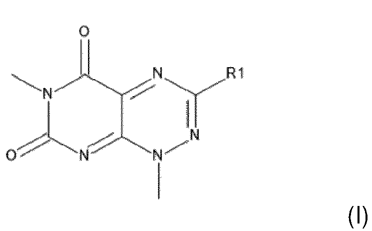
wherein
R1 is selected from p-trifluoromethylphenyl and CH₃;
a compound of formula (II) 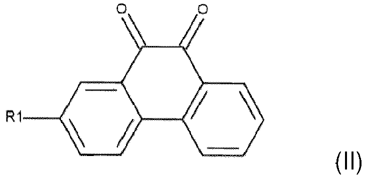 wherein
R1 is selected from the group consisting of H, NO₂, NH₂, and NHCOC(CH₃)₃;
the compounds listed in the table below:
| **Current Name** | **Chemical Name** |
|---|---|
| Ro-08-2750 | 7,10-Dimethyl-2,4-dioxo-2,3,4,10-tetrahydrobenzo[g]pteridine-8-carbaldehyde |
| Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| BVT-948 | 4-Hydroxy-3,3-dimethyl-3,5-dihydro-2H-benzobenzo[g]indole-2,5-dione |
| beta-Lapachone | 2,2-Dimethyl-3,4-dihydro-2H-benzo[h]1-benzopyrane-5,6-dione |
| Dihydrotanshinone | (4R)-4,9-Dimethyl-4,5-dihydrofuro[3,2-c]naphtho[2,1-e]oxepine-1,3-dione |
| NSC-663284 | 6-Chloro-7-[2-(4-morpholinyl)ethylamino]-5,8-dihydroquinoline-5,8-dione |
| Ryuvidine | 2-methyl-5-(4-methylanilino)-1,3-benzothiazole-4,7-dione |
| Propidium Iodide | 3,8-Diamino-5-(3-(diethyl(methyl)ammonio)propyl)-6-phenylphenanthridin-5-ium iodide |
| ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| SEMAPIMOD | N,N'-Bis[3,5-bis[1-(2-amidinohydrazono)ethyl]phenyl]decanediamide |
| Cyanocobalamin | cobalt(3+);[(2R,3S,4R,5S)-5-(5,6-dimethylbenzimidazol-1-yl)-4-hydroxy-2-(hydroxymethyl)oxolan-3-yl][(2R)-1-[3-[(1R,2R,3R,4Z,7S,9Z,12S,13S,14Z,17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl]propanoylamino]propan-2-yl] phosphate;cya nide |
and pharmaceutically acceptable salts thereof.

2. The compound for use according to claim 1, wherein
said compound of formula (I) is selected from 1,6-Dimethyl-3-[4-(trifluoromethyl)phenyl]-1,5,6,7-tetrahydropyrimido[5,4-e][1,2,4]triazine-5,7-dione, 1,3,6-Trimethylpyrimido[5,4-e][1,2,4]triazine-5,7(1H,6H)-dione and pharmaceutically acceptable salts thereof,
or
said compound of formula (II) is selected from the group consisting of 2-Nitrophenanthrene-9,10-dione, N-(9,10-Dioxo-9,10-dihydrophenanthren-2-yl)-2,2-dimethylpropionamide, phenanthrene-9,10-dione, 2-aminophenanthrene-9,10-dione and pharmaceutically acceptable salts thereof.

3. The compound for use according to claim 1 or claim 2, selected from the compounds listed in the table below
| **Current Name** | **Chemical Name** |
|---|---|
| Walrycin B | 1,6-Dimethyl-3-[4-(trifluoromethyl)phenyl]-1,5,6,7-tetrahydropyrimido[5,4-e][1,2,4]triazine-5,7-dione |
| 3-Methyltoxoflavin | 1,3,6-Trimethylpyrimido[5,4-e][1,2,4]triazine-5,7(1H,6H)-dione |
| Ro-08-2750 | 7,10-Dimethyl-2,4-dioxo-2,3,4,10-tetrahydrobenzo[g]pteridine-8-carbaldehyde |
| NSC-23180 | 2-Nitrophenanthrene-9,10-dione |
| SF1670 | N-(9,10-Dioxo-9,10-dihydrophenanthren-2-yl)-2,2-dimethylpropionamide |
| 9,10-Phenanthrenequinone | phenanthrene-9,10-dione |
| PD086278 | 2-aminophenanthrene-9,10-dione |
| Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| BVT-948 | 4-Hydroxy-3,3-dimethyl-3,5-dihydro-2H-benzobenzo[g]indole-2,5-dione |
| beta-Lapachone | 2,2-Dimethyl-3,4-dihydro-2H-benzo[h]1-benzopyrane-5,6-dione |
| Dihydrotanshinone | (4R)-4,9-Dimethyl-4,5-dihydrofuro[3,2-c]naphtho[2,1-e]oxepine-1,3-dione |
| NSC-663284 | 6-Chloro-7-[2-(4-morpholinyl)ethylamino]-5,8-dihydroquinoline-5,8-dione |
| Ryuvidine | 2-methyl-5-(4-methylanilino)-1,3-benzothiazole-4,7-dione |
| Propidium Iodide | 3,8-Diamino-5-(3-(diethyl(methyl)ammonio)propyl)-6-phenylphenanthridin-5-ium iodide |
| ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| SEMAPIMOD | N,N'-Bis[3,5-bis[1-(2-amidinohydrazono)ethyl]phenyl]decanediamide |
| Cyanocobalamin | cobalt(3+);[(2R,3S,4R,5S)-5-(5,6-dimethylbenzimidazol-1-yl)-4-hydroxy-2-(hydroxymethyl)oxolan-3-yl][(2R)-1-[3-[(1R,2R,3R,4Z,7S,9Z,12S,13S,14Z,17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl]propanoylamino]propan-2-yl] phosphate;cyanide |
and pharmaceutically acceptable salts thereof.

4. A pharmaceutical composition for use in the treatment of a SARS-Cov-2 infection in a subject comprising:
i) at least one compound selected from:
a compound of formula (I) 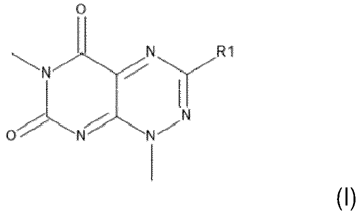 wherein
R1 is selected from p-trifluoromethylphenyl and CH₃;
a compound of formula (II) 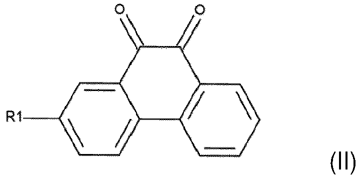 wherein
R1 is selected from the group consisting of H, NO₂, NH₂, and NHCOC(CH₃)₃;
the compounds listed in the table below:
| **Current Name** | **Chemical Name** |
|---|---|
| Ro-08-2750 | 7,10-Dimethyl-2,4-dioxo-2,3,4,10-tetrahydrobenzo[g]pteridine-8-carbaldehyde |
| Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| BVT-948 | 4-Hydroxy-3,3-dimethyl-3,5-dihydro-2H-benzobenzo[g]indole-2,5-dione |
| beta-Lapachone | 2,2-Dimethyl-3,4-dihydro-2H-benzo[h]1-benzopyrane-5,6-dione |
| Dihydrotanshinone | (4R)-4,9-Dimethyl-4,5-dihydrofuro[3,2-c]naphtho[2,1-e]oxepine-1,3-dione |
| NSC-663284 | 6-Chloro-7-[2-(4-morpholinyl)ethylamino]-5,8-dihydroquinoline-5,8-dione |
| Ryuvidine | 2-methyl-5-(4-methylanilino)-1,3-benzothiazole-4,7-dione |
| Propidium Iodide | 3,8-Diamino-5-(3-(diethyl(methyl)ammonio)propyl)-6-phenylphenanthridin-5-ium iodide |
| ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| SEMAPIMOD | N,N'-Bis[3,5-bis[1-(2-amidinohydrazono)ethyl]phenyl]decanediamide |
| Cyanocobalamin | cobalt(3+);[(2R,3S,4R,5S)-5-(5,6-dimethylbenzimidazol-1-yl)-4-hydroxy-2-(hydroxymethyl)oxolan-3-yl][(2R)-1-[3-[(1R,2R,3R,4Z,7S,9Z,12S,13S,14Z,17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl] propa noyla mino] propan-2-yl] phosphate;cya n ide |
and pharmaceutically acceptable salts thereof, and
ii) at least one inert pharmaceutically acceptable excipient.

5. A pharmaceutical composition for use according to claim 4, wherein
said compound of formula (I) is selected from 1,6-Dimethyl-3-[4-(trifluoromethyl)phenyl]-1,5,6,7-tetrahydropyrimido[5,4-e][1,2,4]triazine-5,7-dione, 1,3,6-Trimethylpyrimido[5,4-e][1,2,4]triazine-5,7(1H,6H)-dione and pharmaceutically acceptable salts thereof,
or
said compound of formula (II) is selected from the group consisting of 2-Nitrophenanthrene-9,10-dione, N-(9,10-Dioxo-9,10-dihydrophenanthren-2-yl)-2,2-dimethylpropionamide, phenanthrene-9,10-dione, 2-aminophenanthrene-9,10-dione and pharmaceutically acceptable salts thereof.

6. A pharmaceutical composition for use according to claim 4 or claim 5, wherein said compound is selected from the compounds listed in the table below:
| **Current Name** | **Chemical Name** |
|---|---|
| Walrycin B | 1,6-Dimethyl-3-[4-(trifluoromethyl)phenyl]-1,5,6,7-tetrahydropyrimido[5,4-e][1,2,4]triazine-5,7-dione |
| 3-Methyltoxoflavin | 1,3,6-Trimethylpyrimido[5,4-e][1,2,4]triazine-5,7(1H,6H)-dione |
| Ro-08-2750 | 7,10-Dimethyl-2,4-dioxo-2,3,4,10-tetrahydrobenzo[g]pteridine-8-carbaldehyde |
| NSC-23180 | 2-Nitrophenanthrene-9,10-dione |
| SF1670 | N-(9,10-Dioxo-9,10-dihydrophenanthren-2-yl)-2,2-dimethylpropionamide |
| 9,10-Phenanthrenequinone | phenanthrene-9,10-dione |
| PD086278 | 2-aminophenanthrene-9,10-dione |
| Sodium tanshinone II-A sulfonate | 1,6,6-Trimethyl-10,11-dioxo-6,7,8,9,10,11-hexahydrophenanthro[1,2-b]furan-2-sulfonic acid sodium salt |
| BVT-948 | 4-Hydroxy-3,3-dimethyl-3,5-dihydro-2H-benzobenzo[g]indole-2,5-dione |
| beta-Lapachone | 2,2-Dimethyl-3,4-dihydro-2H-benzo[h]1-benzopyrane-5,6-dione |
| Dihydrotanshinone | (4R)-4,9-Dimethyl-4,5-dihydrofuro[3,2-c]naphtho[2,1-e]oxepine-1,3-dione |
| NSC-663284 | 6-Chloro-7-[2-(4-morpholinyl)ethylamino]-5,8-dihydroquinoline-5,8-dione |
| Ryuvidine | 2-methyl-5-(4-methylanilino)-1,3-benzothiazole-4,7-dione |
| Propidium Iodide | 3,8-Diamino-5-(3-(diethyl(methyl)ammonio)propyl)-6-phenylphenanthridin-5-ium iodide |
| ML120 | 2-[4-[(Z)-[6-ethoxycarbonyl-7-methyl-5-(4-methylsulfanylphenyl)-3-oxo-5H-[1,3]thiazolo[3,2-a]pyrimidin-2-ylidene]methyl]phenoxy]acetic acid |
| SEMAPIMOD | N,N'-Bis[3,5-bis[1-(2-amidinohydrazono)ethyl]phenyl]decanediamide |
| Cyanocobalamin | cobalt(3+);[(2R,3S,4R,5S)-5-(5,6-dimethylbenzimidazol-1-yl)-4-hydroxy-2-(hydroxymethyl)oxolan-3-yl] [(2R)-1-[3-[(1R,2R,3R,4Z,7S,9Z,12S,13S,14Z,17S,18S,19R)-2,13,18-tris(2-amino-2-oxoethyl)-7,12,17-tris(3-amino-3-oxopropyl)-3,5,8,8,13,15,18,19-octamethyl-2,7,12,17-tetrahydro-1H-corrin-21-id-3-yl] propa noyla mino] propan-2-yl] phosphate;cya n ide |

7. A compound for use according to any one of claims 1 to 3 or a pharmaceutical composition for use according to any one of claims 4 to 6, wherein said compound or composition is administered orally.

8. A pharmaceutical composition for use according to any one of claims 4 to 7, in form of a tablet or capsule.

9. A compound for use according to any one of claims 1 to 3 or a pharmaceutical composition for use according to any one of claims 4 to 6, wherein said compound or composition is administered parenterally.

10. A compound or pharmaceutical composition for use according to claim 9, wherein said compound or composition is administered by intraveneous administration or continuous infusion.
